# EUROPÄISCHE PATENTSCHRIFT

(11) **EP 0 873 188 B1**
(45) Veröffentlichungstag und Bekanntmachung des Hinweises auf die Patenterteilung: **27.10.1999**
(21) Anmeldenummer: 96939835.3
(22) Anmeldetag: 18.11.1996
(51) Int. Cl.: B01J 20/26, A61L 15/60, C08F 220/04, C08F 220/58, C08L 33/02, C08L 33/26, C09K 17/22

(54) **WASSERABSORBIERENDE POLYMERE MIT VERBESSERTEN EIGENSCHAFTEN, VERFAHREN ZU DEREN HERSTELLUNG UND DEREN VERWENDUNG**
WATER-ABSORBING POLYMERS WITH IMPROVED PROPERTIES, PROCESS FOR THE PREPARATION AND USE THEREOF
POLYMERES ABSORBANT L'EAU, A PROPRIETES AMELIOREES, LEUR PROCEDE DE PREPARATION ET LEUR UTILISATION

(30) Priorität: 21.11.1995 DE 19543368
(43) Veröffentlichungstag der Anmeldung: 28.10.1998
(73) Patentinhaber: STOCKHAUSEN GmbH & CO. KG, 47805 Krefeld (DE)
(72) Erfinder: HOUBEN, Jochen, D-47906 Kempen (DE)
(74) Vertreter: Klöpsch, Gerald, Dr.-Ing. Patentanwalt
(86) Internationale Anmeldenummer: EP9605074
(87) Internationale Veröffentlichungsnummer: WO9718890

(56) Entgegenhaltungen:
- US-A- 4 618 703
- US-A- 4 906 717
- US-A- 5 154 713
- US-A- 5 314 420

## Beschreibung

Die Erfindung betrifft pulverformige Absorptionsmittel für Wasser und wässrige Flüssigkeiten auf Basis wasserquellbarer, aber nicht wasserlöslicher Polymere. Diese vernetzten Polymerisate auf Basis säuregruppenhaltiger Monomerer werden durch die Verwendung einer speziellen Kombination aus zwei Vorvernetzern und einem Nachvernetzer erhalten und zeigen bislang nicht erreichte Eigenschaftskombinationen aus hoher Retention, hoher Absorption unter Druck, niedrigen löslichen Anteile und einer schnellen Flüssigkeitsaufnahme.

Bei den kommerziell verfügbaren superabsorbierenden Polymeren handelt es sich im wesentlichen um vernetzte Polyacrylsäuren oder vernetzte Stärke/Acrylsäure-Pfropfcopolymerisate, bei denen die Carboxylgruppen teilweise mit Natrium- und/oder Kalium-Ionen neutralisiert sind.

Solche Polymere finden ihren Einsatz z.B. in Hygieneartikeln, die Körperflüssigkeiten wie z.B. Urin aufsaugen können oder in Materialien zur Umhüllung von Kabeln. Dort nehmen sie unter Quellung und Ausbildung von Hydrogelen große Mengen an wässrigen Flüssigkeiten und Körperflüssigkeiten, wie z.B. Urin oder Blut auf. Ferner ist es notwendig, daß die aufgenommene Flüssigkeitsmenge unter dem anwendungstypischen Druck zurückgehalten wird. Im Zuge der technischen Weiterentwicklung der superabsorbierenden Polymere hat sich das Anforderungsprofil an diese Produkte über die Jahre hinweg deutlich verändert. Während in der Entwicklung der Superabsorber zunächst allein das sehr hohe Quellvermögen bei Kontakt mit Flüssigkeit im Vordergrund stand, hat sich später gezeigt, daß es nicht nur auf die Menge der absorbierten Flüssigkeit ankommt, sondern auch auf die Festigkeit des gequollenen Gels. Retention einerseits und Festigkeit des gequollenen Gels andererseits stellen jedoch gegenläufige Eigenschaften dar, wie bereits aus der US 3 247 171 bekannt ist. Das bedeutet, daß Polymere mit besonders hoher Retention nur eine geringe Festigkeit des gequollenen Gels aufweisen mit der Folge, daß das Gel unter einem angewendeten Druck (z.B. Körperdruck) deformierbar ist und die weitere Flüssigkeitsaufnahme verhindert. Diese spezifische Absorptionseigenschaft, die im angelsächsichen Sprachgebrauch "absorption under pressure" (AUP) heißt, wird z.B. in der US 5 314 420 beschrieben.
Im Zuge der gestiegenen Anforderungen an Superabsorber im Hygienebereich hat es sich herausgestellt, daß die ursprüngliche Belastung von 21 g/cm2 (0,3 psi) nicht mehr dem erwünschten Eigenschaftsstandard entspricht, wie er für Inkontinenzprodukte bzw. für Windelkonstruktionen mit niedrigem Fluffgehalten und hohen Mengen Superabsorber erforderlich ist. Demzufolge werden heute Druckbelastungen von 49 g/cm2 (0,7 psi) gefordert.

Dem Fachmann sind zwar Methoden bekannt, wie z.B. Produkte mit einer hohen Retention oder einer hohen Absorption oder niedrigen löslichen Anteilen oder mit einer schnellen Wasseraufahme hergestellt werden können, aber die gleichzeitige Einstellung aller dieser vier positiven Eigenschaften war mit den bisher bekannten Rezepturen nicht möglich. So ist es dem Fachmann wohl bekannt, daß eine Erhöhung der Vernetzerkonzentration Produkte mit niedrigen löslichen Anteilen ergibt, gleichzeitig führt dies aber zu Produkten mit niedriger Retention. Umgekehrt führt die Absenkung der Vernetzerkonzentration zwar zu Produkten mit hohen Retentionen aber ebenso zu hohen löslichen Anteilen und bedingt durch das Gelblocking zu einer langsamen Wasseraufnahme.

Der Versuch in der US Re 32,649 Produkte mit hohem Gelvolumen, hoher Gelstärke und niedrigen löslichen Anteilen zu erhalten, führte nur bei Verwendung des das carcinogene Acrylamid abspaltenden Vernetzers Methylenbisacrylamid zu den erwünschten Produkteigenschaften. Eine Verwendung von solchen Produkten in Hygieneartikeln ist deshalb nicht möglich.

Neben einem hohen Niveau der Retention und Flüssigkeitsaufnahme unter Druck müssen Superabsorber einen möglichst geringen Anteil an löslichen Polymerketten enthalten. Diese entstehen aufgrund unvollkommener Vernetzung während der Polymerisation. Im Anwendungsfall werden diese löslichen Anteile im gequollenen Polymerkörper nicht vollständig zurückgehalten. Dies führt zu einer Leistungsverminderung des Superabsorbers, da die Flüssigkeit in der Windel nicht mehr gleichmäßig verteilt wird und kann darüber hinaus im Extremfall zu einem Austreten dieser löslichen Anteile aus der Windelkonstruktion, verbunden mit einem schleimigen Gefühl auf der Haut führen. Nach der Lehre der EP 312 952 ist es für eine gute Stabilität des gequollenen Gels, sowie für eine hohe Aufnahmegeschwindigkeit und eine hohe Wasseraufnahme unabdingbar, daß das Polymer niedrige lösliche Anteile aufweist. Als Grenzwert für niedrige lösliche Anteile wurden z.B. in der US Re 32,649 17% nach 16 Stunden angegeben, was aufgrund der inzwischen vonstatten gegangenen Entwicklung zu dünneren Windeln mit höherem Superabsorberanteil pro Windel als zu hoch angesehen werden muß. Ein Wert von 12% oder besser noch 10% nach 16 Stunden sollte bei modernen Superabsorbern realisiert werden.

Eine weitere Anforderung an Superabsorber in solchen Sanitärartikeln mit erniedrigtem Fluff- und erhöhtem Superabsorberanteil ist die Eigerschaft, Flüssigkeit bereits im Moment des Auftretens sehr schnell aufzunehmen, da die Pufferwirkung des Fluffanteils deutlich verringert ist. Die US 5 314 420 lehrt eine Methode zur Herstellung eines schneller saugenden Superabsorbers durch die Zugabe von Natriumcarbonat. Hierbei tritt offenbar nur eine physikalische Strukturveränderung der Superabsorber ein, die eine schnellere Sauggeschwindigkeit bewirkt, die anderen Grundeigenschaften des Polymers jedoch nicht verbessert. Eine Behandlag der Oberfläche mit einem tensidartigen Material zur Erhöhung der Wasseraufnahmegeschwindigkeit, wie in der WO 93/24153 beschrieben, scheitert an dem für die Praxis wichtigen Rewetverhalten, d.h. der Eigenschaft des superabsorbierenden Polymers einmal aufgenommene Flüssigkeit dauerhaft zu speichern. Dies wird durch das Vorhandensein von Tensiden empfindlich gestört.

Der WO 94/09043 liegt die Aufgabenstellung zugrunde, neue superabsorbierende Polymere mit einem für wässrige Flüssigkeiten erhöhten Aufnahmevermögen, auch unter Druckbelastung bereitzustellen. Sie beschreibt als Lösung dieser Aufgabe doppelt vernetzte Superabsorber, deren Herstellung in der ersten Stufe die Vorvernetzung während der Polymerisation mit Methylenbisacrylamid, Bis(acrylamido)essigsäure, Allylacrylat, Allylmethacrylat, Estern bzw. Amiden mit endständigen Vinyl- und Allylfunktionen oder hoch ethoxyliertem Trimethylolpropantriacrylat vorsieht und in der zweiten Stufe die entstandenen Polymerpartikel an der Oberfläche mit einem Vernetzer beschichtet und dann vernetzt. Bei diesem, an sich bereits bekannten Verfahren, sind die bevorzugten Oberflächenvernetzer Polyhydroxyverbindungen, die zusammen mit Wasser oder Wasser/Lösungsmittelgemischen aufgebracht und bei erhöhten Temperaturen (175-230°C) zur Reaktion gebracht werden, nachdem zuvor die Feuchtigkeit des Polymergels der ersten Stufe teilweise entfernt wurde.

Durch die Kombination eines der genannten primären Vernetzer mit den sekundären Oberflächenvernetzern sollen angeblich einzigartige Produkteigenschaften bezüglich der Retention und der Flüsssigkeitsaufnahme unter Druck erreicht werden, wodurch die vorteilhafte Anwendung in Hygieneartikeln, bei denen die absorbierenden Polymere beträchtliche Mengen an Flüssigkeit aufsaugen und auch unter Druckbelastung zurückhalten müssen, gegeben ist. Die für moderne Windelkonstruktionen wichtige Forderung nach einer schnellen Flüssigkeitsaufnahme wird durch diese Produkte aber nicht erfüllt.

Dic WO 93/21237 beschreibt superabsorbierende Polymere, die mit ungesättigten Estern von Polyalkylglykolen vernetzt sind und die in einem Nacherhitzungsprozeß eine Eigenschaftsverbesserung bezüglich Retention und Flüssigkeitsaufnahme unter niedrigem Druck 21 g/cm2 (0.3 psi) auf jeweils 25 g/g erlangen. Ethoxyliertes Trimethylolpropantriacrylat ist der bevorzugte Vernetzer, wobei die Anzahl der EO-Einheiten pro Polyglykolkette zwischen 2 und 7 liegen kann. Gemäß den Ausführungen in dieser Schrift führt die Verwendung von nicht oder nur gering ethoxyliertem Trimethylolpropantriacrylat zu wesentlich schlechteren Eigenschaften der damit vernetzten Superabsorber. Die beschriebenen Produkte erfüllen nicht die heutzutage gestellten Anforderungen bezüglich der Absorption unter höherem Druck bei 49 g/cm2 (0.7 psi). In der Abbildung 13 auf Seite 8/8 der WO 93/21237, die den Verlauf der Flüssigkeitsaufnahme unter Druck für verschiedene Druckbelastungen darstellt, zeigt sich ganz klar die Schwäche der dort beschriebenen Polymere, deren Meßwerte von ca. 18 g/g im interessanten Druckbelastungsbereich von 63 g/cm2 (0.9 psi) völlig unbefriedigend sind. Dies gilt um so mehr, als die Meßwerte an einer völlig unüblichen Siebfraktion von 300 - 600 µm ermittelt wurden, die per se höhere Meßwerte ergibt, als die praxisübliche Siebfraktion von 150 - 800 µm.

Die US 5 314 420 hatte es sich zur Aufgabe gestellt, Produkte zur Verfügung zu stellen, die in der Lage sind, die Flüssigkeit sehr schnell aufzunehmen. Dies gelingt durch Zusatz eines carbonathaltigen Blähmittels zur Monomerlösung und anschließender Nachvernetzung des gebildeten Polymers. In dieser Schrift wird die Möglichkeit angedeutet, mehrere bevorzugte Vernetzer zu kombinieren, eine Problemlösung zur gleichzeitigen Erhöhung von Retention, Absorption unter Druck und Aufnahmegeschwindigkeit sowie Reduzierung von löslichen Anteilen wird nicht gegeben.

Mit den bislang hergestellten Produkten ließen sich die Eigenschaften hohe Retention, hohe Absorption unter Druck und schnelle Flüssigkeitsaufnahme bei gleichzeitig niedrigen löslichen Anteilen noch nicht kombinieren.

Ein weiteres Problem bei der Herstellung von superabsorbierenden Polymeren ist die relativ schlechte Löslichkeit vieler Standardvernetzer in der wässrigen Monomerlösung bei den niedrigen Temperaturen des Polymerisationsstarts von z.B. deutlich unter 20°C. Bei diesen Temperaturen wird die wässrige Monomerlösung bei Verwendung der üblichen Vernetzer, wie Trimethylolpropantriacrylat, Diethylenglykoldiacrylat, Tetraethylenglykoldimethacrylat oder Allylmethacrylat, um nur einige Beispiele zu nennen, trüb. Diese trübe Lösung weist daraufhin, daß die Vernetzer nicht homogen gelöst vorliegen, was bei der nachfolgenden Polymerisation zu einer unregelmäßigen Vernetzung und damit zu einem minderwertigen Produkt führt.

Um diese Problematik zu umgehen schlägt die DE-OS 41 38 408 vor, zu der Monomerlösung ein nicht einpolymerisierbares Tensid zuzusetzen, um den ansonsten schlecht löslichen Vernetzer gleichmäßiger zu verteilen. Dieses Vorgehen hat aber zwei entscheidene Nachteile. Zum Einen schäumt die tensidhaltige Monomerlösung beim Ausblasen mit Stickstoff derart stark, daß der Ausblasevorgang zur Entfernung des störenden Sauerstoffs aus der Monomerenlösung länger dauert, als ohne Tensidzusatz. Zum Anderen begünstigen die Tenside im Polymer den Wiederaustritt des aufgenommenen Wassers, was zu der ungewünschten Erscheinung des Rewets führt und das Produkt für einen Einsatz in Hygieneartikeln disqualifiziert, da es aufgenommene Flüssigkeit wieder zu leicht abgibt.

Eine andere Lösung des beschriebenen Problems lehrt die WO 93/21237, in der (Meth)acrylsäureester von hochethoxylierten Polyglykolen, wobei ethoxyliertes Trimethylolpropan bevorzugt ist, als Vernetzer gewählt werden. Diese Produkte sind je nach Oxethylierungsgrad in der Monomerlösung löslich und damit gleichmäßig verteilt, was demzufolge Produkte mit den erwünscht niedrigen löslichen Anteilen ergibt. Nachteilig an diesen Vernetzern ist es jedoch, daß beim nachvernetzten Produkt keine Retentionen von über 30 g/g, sowie die Absorption unter Druck (21 g/cm²) nicht über einen Wert von 28 g/g (Tabelle 1) hinauskommt. Die Verwendung von nicht oder nur gering ethoxylierten Vernetzern ist nicht möglich, da sie in diesem System schlechte Werte, speziell bei den löslichen Anteilen hervorrufen. Darüber hinaus gibt die Schrift keine Lösung bezüglich einer Verbesserung der Saugkraft des Absorbers an.

Aufgabe der Erfindung ist es daher, für die Herstellung von superabsorbierenden Polymeren Vernetzer oder Vernetzerkombinationen bereitzustellen, die ohne weitere Hilfsstoffe in der Monomerenlösung löslich sind und die bei nachvernetzten Polymeren zu guten Retentionen und Absorptionen unter Druck führen und gleichzeitig niedrige lösliche Anteile von weniger als 12% (16h-Wert) ergeben und damit Produkte mit schneller Wasseraufnahme bereitzustellen. Gleichzeitig sollte auf Vernetzer verzichtet werden, die wie z.B. Methylenbisacrylamid das carcinogene Acrylamid abspalten oder die wie z.B. Ethylenglykoldimethacrylsäureester eine Sensibilisierung hervorrufen können.

Es wurde überraschenderweise gefunden, daß Superabsorber auf Basis säuregruppengruppenhaltiger Monomeren, die mittels einer Kombination aus zwei verschiedenen, an sich bekannten Vorvernetzern I und II
I. CH₂=CR⁵-CO-(OCHR³-CHR³)_{z}-CH₂-CR⁵=CH₂
II. R¹-[O(CHR³-CHR³O)ᵤ-CO-R₂]ₓ
   mit
   R¹: mehrwertiges C₂₋₁₀-Alkyl,
   R²: linear oder verzweigt C₂₋₁₀-Alkenyl,
   R³: H, CH₃, C₂H₅,
   R⁵: H, CH₃,
   x: 2-6,
   u: 0-15,
   z: 3-20
und einer anschließenden Nachvernetzung mit einem Nachvernetzer hergestellt werden, zu Produkten führt, die eine Retention von über 30 g/g, einer Absorption unter Druck von über 20 g/g, löslichen Anteilen (16h) von unter 12 % und gleichzeitig noch eine schnelle Flüssigkeitsaufnahme, ausgedrückt durch eine Schwellgeschwindigkeit von unter 40 sec aufweisen.

Der Vorvernetzer I, ein (Meth)acrylsäureester eines Polyglykolmono(meth)allylethers, enthält eine (Meth)allylfunktion, eine (Meth)acrylsäureesterfunktion und eine zwischen diesen beiden Funktionen eingeschobene hydrophile Kette, die aus aus mindestens drei, vorzugsweise 5 bis 20, besonders bevorzupt 8-12 Glykoleinheiten besteht. Als Glykoleinheiten kommen sowohl Ethylenglykol als auch Propylenglykoleinheiten, einzeln oder gemischt in Frage, wobei bei Mischungen sowohl Random- als auch Blockalkoxylate in Frage kommen. Die Verwendung von gemischten Ethylenglykol/Propylenglykolketten, als auch reinen Propylenglykolketten ist möglich, wobei reine Polyethylenglykolketten bevorzugt werden.

Die beschriebenen Vorvernetzer vom Typ I lassen sich beispielsweise nach der in der US 4 618 703 beschriebenen Prozedur oder durch Direktveresterung mit einem Überschuß Acrylsäure bzw. Methacrylsäure (Beispiele 4 9) herstellen. Die so erhaltenen Ester können roh, d.h. in Gegenwart des eingesetzten Katalysators und der im Überschuß eingesetzten (Meth)acrylsäure gelagert und weiterverarbeitet werden. Für höhere Reinheitsanforderungen oder zwecks längerer Lagerung ist es auch möglich, die Ester wie in der US 4618 703 beschrieben, zu waschen und destillativ von leichtsiedenden Bestandteilen zu reinigen. Der Veresterungsgrad sollte über 90, besser über 95% liegen, da freie Hydroxylfünktionen zunächst nichts zur Verbesserung der anwendungstechnischen Eigenschaften beitragen und bei einer angeschlossenen Nachvernetzung zur Absenkung der Retention führen.

Beim Vorvernetzer II, einem (Meth)acrylsäureester von Polyhydroxyverbindungen, handelt es sich um mehrfachfunktionelle Alkohole, deren Alkoholfunktionen zu (Meth)acrylsäureestern umgesetzt wurden. Als solche Polyhydroxyverbindungen kommen z.B. Trimethylolpropan, Ethylenglykol, Propylenglykol, Glycerin, Pentaerythrit oder deren ethoxylierten Homologe wie z.B. Polyethylenglykol in Frage. Eingeschlossen sind auch solche Ester, bei denen ein Teil der Hydroxylgruppen unverestert geblieben ist, wie dies bei technischen Produkten der Fall sein kann. Insbesondere ist es auch möglich, als Vernetzer II sonst in der Monomerlösung schlecht lösliche (Meth)acrylsäureester, wie Trimethylolpropantriacrylat, Trimethylolpropan3EO-triacrylat, Ethylenglykoldiacrylat, Diethylenglykoldimethacrylat einzusetzen, da diese vom Vernetzer I in Lösung gehalten werden.

Die Vorvernetzer vom Typ II sind kommerziell erhältlich. Das in den Beispielen verwendete Craynor CN 435 ist der Triacrylsäureester des 15-EO-Trimethylolpropans, das verwendete Craynor SR 351 ist der Triacrylsäureester von Trimethylolpropan. Beides sind Produkte der Cray-Valley-Company.

Der Vorvernetzer I wird mit 40-90, bevorzugt 40-80, besonders bevorzugt 60-80 Molprozent bezogen auf die Mischung der Vernetzer und der Vorvernetzer II mit 10-60, bevorzugt 20-60, besonders bevorzugt 20-40 Molprozent bezogen auf das Vernetzergemisch eingesetzt. In Bezug auf die ungesättigte saure Monomerkomponente wird diese Vorvernetzerkombinationen in Konzentrationen von 0,1-2%, vorzugsweise 0,3 - 1,0 Gew% eingesetzt.

Beide Vorvernetzertypen für sich alleine sind dem Durchschnittsfachmann für die Herstellung von vernetzten Polyacrylaten bekannt. Insbesondere die Möglichkeit der Verwendung von (Meth)acrylsäureestern von Polyglykolmonoallylethern ist in den US-Patenten 4 076 663, 4 654 039, 4 906 717, 5 154 713 und 5 314 420 erwähnt. Allerdings ist aus der US 4 654 039, US 5 154 713, US 5 314 420 und US 4 076 663 die Möglichkeit der vorteilhaften Kombination des Vorvernetzertyps I mit dem Vorvernetzertyp II nicht zu entnehmen. Lediglich in der US 4 906 717 wird die Möglichkeit der Kombination mehrerer Vernetzertypen, unter denen in einer umfangreichen Liste auch die erfindungsgemäßen sind, erwähnt, ohne die Vorteile einer solchen Kombination offenzulegen oder Ausführungsbeispiele zu geben. Insbesondere sind die Vorteile dieser Vernetzerkombination in der Kombination mit der erfindungsgemäßen Nachvernetzung nicht erwähnt und erkannt worden.

Die beschriebenen Vorvernetzerkombination zeigt den überraschenden Vorteil, daß auch schwer lösliche Vernetzerkomponenten II durch die Anwesenheit von I solubilisiert werden und dadurch ihre Wirksamkeit voll entfalten können, ohne daß das Rewet-Verhalten durch das Vorhandensein nicht einpolymerisierter Tenside wie bei der DE 41 38 408 negativ beeinflußt wird.

Die erfindungsgemäßen Superabsorber werden durch das Verfahren der nachträglichen Oberflächenvernetzung in ihrem Eigenschaftsprofil verbessert, insbesondere auch in ihrer Flüssigkeitsaufnahme unter Druck, da das bekannte Phänomen des "gel blocking" unterdrückt wird, bei dem angequollenen Polymerteilchen miteinander verkleben und eine weitere Flüssigkeitsaufnahme und Flüssigkeitsverteilung in der Windel behindern. Während der Nachvernetzung werden die Carboxylgruppen der Polymermoleküle an der Oberfläche der Superabsorberteilchen mit Vernetzungsmitteln unter erhöhter Temperatur vernetzt. Erfindungsgemäße Verfahren zur Nachvernetzung sind in mehreren Schriften, wie z.B. der DE 40 20 780 ,der EP 317 106 und der WO 94/9043 beschrieben. Die dem Fachmann z.B aus der US 5 314 420, Seite 8, Zeile 3-45 bekannten Nachvernetzungsmittel können erfindungsgemäß alle vorteilhaft in Kombination mit der Vorvernetzerkombination I und II eingesetzt werden. Diese Verbindungen enthalten in der Regel mindestens zwei funktionelle Gruppen. Dabei sind Alkohol-, Amin-, Aldehyd- Glycidyl- und Epichlorfunktionen bevorzugt, wobei auch Vernetzermoleküle mit mehreren verschiedenen Funktionen denkbar sind. Bevorzugt wird eines der folgenden Nachvernetzungsmittel eingesetzt: Ethylenglykol, Diethylenglykol, Triethylenglykol, Polyethylenglykol, Glycerin, Polyglycerin, Propylenglykol, Diethanolamin, Triethanolamin, Polypropylenoxyd, Blockcopolymere aus Ethylenoxyd und Propylenoxyd, Sorbitanfettsäureester, ethoxylierte Sorbitanfettsäureester, Trimethylolpropan, ethoxyliertes Trimethylolpropan, Pentaerythrit, ethoxyliertes Pentaerythrit, Polyvinylalkohol, Sorbit, Ethylencarbonat, Proypylencarbonat und Polyepoxide wie etwa Ethylenglykoldiglycidylether. Besonders bevorzugt wird mit Ethylencarbonat als Nachvernetzungsmittel gearbeitet. Das Nachvernetzungsmittel wird in einer Menge von 0,01 bis 30 Gewichtsprozent, bevorzugt 0,1-10 Gewichtsprozent, besonders bevorzugt 0,1-1 Gewichtsprozent bezogen auf das nachzuvernetzende Polymer eingesetzt.

Das erfindungsgemäße Polymerisationsverfahren kann durch verschiedene Bedingungen initiiert werden, wie z.B. durch Bestrahlung mit radioaktiven, elektromagnetischen oder ultravioletten Strahlen oder durch Redoxreaktion zweier Verbindungen, wie z.B. Natriumhydrogensulfit mit Kaliumpersulfat oder Ascorbinsäure mit Wasserstoffperoxid. Auch der thermisch ausgeloste Zerfall eines sogenannten Radikalstarters wie beispielsweise Azobisisobutyronitril, Natriumpersulfat, t-Butylhydroperoxid oder Dibenzoylperoxid ist als Polymerisationsstart einsetzbar. Ferner ist die Kombination von mehreren der vorgenannten Methoden denkbar. In der vorliegenden Erfindung wird vorzugsweise der Polymerisationsstart durch die Redoxreaktion zwischen Wasserstoffperoxid und Ascorbinsäure ausgelöst und durch den thermisch initiierten Zerfall von Natriumpersulfat und/oder 2,2'-Azobis(2-methyl-propionamid)dihydrochlorid vollendet.

Für die Polymerisation der erfindungsgemäßen Superabsorber kommen mehrere Verfahren in Frage, z.B. Massepolymerisation, Lösungspolymerisation, Spraypolymerisation, inverse Emulsionspolymerisation und inverse Suspensionspolymerisation. Bevorzugt wird die Lösungspolymerisation in Wasser als Lösungsmittel durchgeführt. Die Lösungspolymerisation kann kontinuierlich oder diskontinuierlich erfolgen. Die Patentliteratur weist ein breites Spektrum an Variationsmöglichkeiten hinsichtlich der Konzentrationsverhältnisse, Temperaturen, Art und Menge der Initiatoren als auch der Nachkatalysatoren auf. Typische Verfahren sind in den folgenden Patentschriften beschrieben, die hiermit durch Verweis Bestandteil des erfindungsgemäßen Herstellverfahrens werden sollen: US 4 286 082, DE 27 06 135, US 4 076 663, DE 35 03 458, DE 40 20 780, DE 42 44 548, DE 43 23 001, DE 43 33 056, DE 44 18 818.

Die erfindungsgemäß einzusetzenden, Säuregruppengruppen enthaltenden ungesättigten Monomere sind beispielsweise Acrylsäure, Methacrylsäure, Crotonsäure, Isocrotonsäure, Maleinsäure, Fumarsäure, Itaconsäure, Vinylsulfonsäure, 2-Acrylamido-2-methyl-l-propansulfonsäure, Vinylessigsäure, Methallylsulfonsäure sowie deren Alkali- und/oder Ammoniumsalze. Zur Modifizierung der Polymereigenschaften können optional bis zu 40 Gew.% weitere Comonomere, bezogen auf die sauren Monomere, wie etwa Acrylamid, Methacrylamid, und oder deren Salze verwendet werden. Ferner ist es auch möglich, daß Kombinationen der oben erwähnten Monomeren eingesetzt werden, sowie Kombinationen der oben erwähnten Monomeren mit nichtionischen, hydrophilen Monomeren, wie z.B (Meth)allylalkohol, und die Mono(Meth)acrylsäureester von mehrwertigen Alkoholen oder Oxethylaten. Auch können (Meth)acrylnitril, Vinylpyrrolidon, Hydroxyethylacrylat und Vinylacetamid verwendet werden. Bevorzugt werden jedoch Acrylsäure, Methacrylsäure bzw. deren Alkali- oder Ammoniumsalze eingesetzt, besonders bevorzugt wird Acrylsäure und deren Natrium- und/oder Kaliumsalz eingesetzt.

Die Neutralisation der sauren Monomeren in dem erfindungsgemäßen Polymerisationsverfahren kann auf verschiedene Art und Weise durchgeführt werden. Zum einen kann die Polymerisation entsprechend der Lehre der US 4 654 039 direkt mit den sauren Monomeren durchgeführt werden, wobei die Neutralisation dann anschließend im Polymergel erfolgt. Zum anderen und bevorzugt werden die sauren Monomerebestandteile vor der Polymerisation zu 25-95%, bevorzugt 50-80% neutralisiert und liegen dann bereits bei Beginn der Polymerisation als Natrium-, und/oder Kalium und/oder Ammoniumsalze vor. Zur Neutralisation werden bevorzugt solche Basen verwendet, die keinen negativen Einfluß auf die spätere Polymerisation haben. Bevorzugt wird Natron- oder Kalilauge und oder Ammoniak, besonders bevorzugt Natronlauge, genommen, wobei ein Zusatz von Natriumcarbonat, Kaliumcarbonat oder Natriumbicarbonat einen zusätzlichen positiven Effekt ausüben kann, wie die US 5 314 420 und die US 5 154 713 lehren. Diese teilneutralisierte Monomerlösung wird vor dem Start der Polymerisation bei der adiabatischen Lösungspolymerisation auf eine Temperatur von unter 30°C, bevorzugt unter 20°C heruntergekühlt. Bei den anderen erwähnten Verfahren sind nach dem Stand der Technik auch andere Temperaturen bekannt und üblich.

Die erfindungsgemäßen Polymerisate können wasserlösliche Polymere als Pfropfgrundlage in Mengen bis zu 30 Gew.%, bezogen auf die Summe der vorhandenen Monomere, enthalten. Dazu zählen unter anderem teil- oder vollverseifte Polyvinylalkohole, Stärke oder Stärkederivate, Cellulose oder Cellulosederivate, Polyacrylsäuren, Polyglykole oder deren Gemische. Die Molekulargewichte der als Pfropfgrundlage zugesetzten Polymere müssen an die Gegebenheiten der Polymerisationsbedingungen angepaßt sein. So kann es z.B. im Falle einer wäßrigen Lösungspolymerisation aus Gründen der Viskosität der Polymerisatlösung erforderlich sein, nur niedrig- oder mittelmolekulare Polymere einzusetzen, wohingegen bei der Suspensionspolymerisation dieser Faktor eine untergeordnete Rolle spielt.

Neben Polymerisaten, die durch vernetzende Polymerisation teilneutralisierter Acrylsäure zu erhalten sind, werden bevorzugt solche verwendet, die zusätzliche Anteile von pfropfpolymerisierter Stärke oder von Polyvinylalkohol enthalten.

Die sauren Monomeren sind im Endprodukt mindestens zu 25 Mol% und bevorzugt zu mindestens 50 Mol% und besonders bevorzugt zu 50 bis 80 Mol% neutralisiert. Die Neutralisation wird entweder durch die Zugabe der entsprechenden Alkali- bzw. Ammoniumhydroxyde oder mit den entsprechenden Carbonaten- oder Hydrogencarbonaten ausgeführt. Dabei kann die teilweise Neutralisation bereits schon bei der Herstellung der Monomerlösung, also vor der Polymerisation oder wie in der US 4 654 039 beschrieben erst an dem fertigen Polymerisat ausgeführt werden. Vorzugsweise wird die teilweise Neutralisation bereits vor der Polymerisation durchgeführt.

Das resultierende Polymer wird getrocknet, gemahlen und auf die für die Einarbeitung in Wegwerfwindeln, Kabelisolierungen oder anderen Produkten jeweils anwendungstechnisch günstige Kornfraktion abgesiebt und dann der Nachvernetzungsreaktion zugeführt. In manchen Fällen hat es sich jedoch auch bewährt, die Nachvernetzer bereits vor der Trocknung des Polymergels bzw. vor der Zerkleinerung des teilweise oder überwiegend getrockneten Polymers zuzufügen. Eine erfindungsgemäß durchzuführende Nachvernetzung wird z.B. in der US 4 666 983 und der DE 40 20 780 beschrieben. Der Zusatz der Nachvernetzer erfolgt häufig vorteilhafterweise auch in Form einer Lösung in Wasser, organischen Lösemitteln oder deren Mischungen, insbesondere dann, wenn geringe Mengen an Nachvernetzungsmittel angewandt werden. Geeignete Mischaggregate zum Aufbringen des Nachvernetzungsmittels sind z.B. Patterson-Kelley-Mischer, DRAIS-Turbulenzmischer, Lödigemischer, Ruberg-Mischer, Schneckenmischer, Tellermischer und Wirbelschichtmischer, sowie kontinuierlich arbeitende senkrechte Mischer in denen das Pulver
mittels rotierender Messer in schneller Frequenz gemischt wird (Schugi-Mischer). Nachdem der Nachvernetzer mit dem vorvernetzten Polymer vermischt worden ist, wird zur Durchführung der Nachvernetzungsreaktion auf Temperaturen von 120 bis 250 °C, bevorzugt auf 135 bis 200°C und besonders bevorzugt auf 150 bis 185°C erhitzt. Die Zeitdauer der Nacherhitzung ist durch den Punkt begrenzt, bei dem das gewünschte Eigenschaftsprofil des Superabsorbers infolge von Hitzschädigung wieder zerstört wird.

Für die Verarbeitung der Superabsorber werden je nach Anwendungsfall unterschiedliche Siebfraktionen eingesetzt, so z.B. für Windeln zwischen 100 und 1000 mm, bevorzugt zwischen 150 und 850 mm. Diese Kornfraktion wird im allgemeinen durch Mahlung und Siebung vor der Nachvernetzung hergestellt.

Die erfindungsgemäßen hydrophilen Superabsorber finden überall dort ihre Verwendung, wo wäßrige Flüssigkeiten absorbiert werden müssen. Dazu gehören beispielsweise die bereits bekannten Anwendungen für diese Produkte in Hygieneartikeln in Form von Windein für Kleinkinder und Inkontinenzprodukten für Erwachsene, in Damenbinden, in Wundpflastern, in Lebensmittelverpackungen, im Agrarbereich bei der Pflanzenaufzucht, in Kabelisolierungen, in absorbierenden Flächengebilden aus Papier, wasserlöslichen Polymeren und thermoplastischen Kunststoffen und Schäumen, sowie als Wirkstoffträger mit der Aufgabe der zeitlich verzögerten Freisetzung an die Umgebung.

In den folgenden Beispielen wird die Herstellung erfindungsgemäß zu verwendender Vernetzer und die Herstellung und Eigenschaften der erfindungsgemäßen Polymerisate erläutert, in dem Kapitel Prüfmethoden werden die Vorschriften zur Bestimmung der Eigenschaften der Superabsorber beschrieben.

### Prüfmethoden

### 1. Retention (TB)

Die Retention wird nach der in der EP 514 724 (Seite 4, Zeilen 6-22) beschriebenen Methode gemessen.

### 2. Flüssigkeitsaufnahme unter Druck (AUP)

Die Flüssigkeitsaufnahme unter Druck (AUP bei 0,3 und 0,7 Psi, entsprechend 21 g/cm² bzw. 49 g/cm²) wird nach der in der US 5 314 420 Seite 9, Zeile 28 ff beschriebenen Methode bestimmt, wobei als Messflüssigkeit 0,9%ige Kochsalzlösung verwendet wird.

### 3. Lösliche Anteile (LA)

Die Löslichen Anteile (1h und 16h) werden wie in der US 4 654 039 beschrieben bestimmt, mit der Ausnahme, daß als Testflüssigkeit statt synthetischem Urin eine 0,9%-ige Kochsalzlösung genommen wird.

### 4. Schwellgeschwindigkeit (SG)

Die Schwellgeschwindigkeit(SG) des Polymers wird mittels der folgenden Methode bestimmt: Zwanzig (20) Gramm einer synthetischen Urin-Lösung (hergestellt nach US 4 654 039) wird in ein schmales Becherglas eingewogen. Ein (1) Gramm des zu testenden Materials wird in eine breite (5 cm Durchmesser) zylindrische Schale zentral in der Mitte eingewogen. Das Pulver mit den superabsorbierenden Eigenschaften wird durch leichtes Schütteln gleichmäßig über den Boden der Schale verteilt. Die synthetische Urin-Lösung wird mittels eines 1 cm über dem Boden der Schale endenden Trichters mit einem Guß zugegeben und damit die Zeitmessung in Gang gesetzt. Die Zeitmessung wird beendet, sobald keine freie Flüssigkeit mehr zu erkennen ist.

### Beispiele

### Beispiel 1: (Umsetzung von Allylalkohol mit 5 mol Ethylenoxid)

464,8 g Allylalkohol (Merck) werden zusammen mit 4 g Natriummethylatlösung (25%-ig) in einem 51-Autoklaven mit Hubrührer vorgelegt und durch fünfmaliges Aufdrücken von Stickstoff bis auf 5 bar und anschließendes Entspannen auf 1 bar sauerstoffrei gemacht. Unter weiterem Durchleiten von Stickstoff wird der Reaktorinhalt zunächst auf 70°C aufgeheizt, wobei das mit dem Katalysator eingesetzte Methanol entfernt wird. Anschließend wird der Reaktor verschlossen, auf 140°C aufgeheizt und bei einem Gesamtdruck von 3-6 bar 1760 g Ethylenoxid innerhalb 30 Minuten aufgedrückt. Als Produkt werden 2210 g hellgelbe Flüssigkeit erhalten. Kennzahlen siehe Tabelle 1.

### Beispiel 2 (Umsetzung von Allylalkohol mit 10 mol Ethylenoxid)

290,5g Allylalkohol (5 mol) werden zusammen mit 2,5 g fester KOH (85%-ig) vorgelegt und wie im Beispiel 1 beschrieben inertisiert. 2200 g Ethylenoxid (50 mol) werden innerhalb einer Stunde bei 4-6 bar und einer Reaktionstemperatur von 140°C aufgedrückt. Als Produkt werden 2480 g hellgelbe Flüssigkeit erhlalten. Kenndaten siehe Tabelle 1.

### Beispiel 3 (Umsetzung von Allylalkohol mit 20 mol Ethylenoxid)

1495,8 g (3 mol) des Produktes aus Beispiel 2 werden im fünf-Liter-Autoklav vorgelegt, entsprechend der Prozedur aus Beispiel 1 inertisiert und innerhalb 1 Stunde bei 140°C mit 1320 g (30 mol) Ethylenoxid umgesetzt. Man erhält 2805 g eines gelb gefärbten Feststoffs. Kenndaten siehe Tabelle 1.

**Tabelle 1**

| Kennzahlen der hergestellten Ethoxylate | | | |
|---|---|---|---|
| Produkt | Beispiel 1 | Beispiel 2 | Beispiel 3p |
| OH-Zahl | 204 mg KOH/g | 118 mg KOH/g | 53 mg KOH/g |
| Jod-Zahl (Wijs) | 91 mg KOH/g | 53 mg KOH/g | 27 mg KOH/g |
| Allylalkohol (GC) | 2030 ppm | 144 ppm | 14 ppm |
| Festpunkt | -15°C | 3°C | 29°C |
| Jodfarbzahl | 3 | 7 | - |
| Dichte | 1,054 g/ml | 1,085 g/ml | - |
| Viskosität RV 2/10 | 18 mPas (20°C) | 68 mPas (20°C) | - |

### Beispiel 4: (Acrylsäureesters von 10-EO-Allylalkohol AAA-10):

245 g (0,726 mol) 10-EO-Allylalkohol aus Beispiel 2 werden zusammen mit 155,6 g (2,16 mol) Acrylsäure und 0,8 g p-Methoxyphenol bei 20°C gerührt, bis sich das p-Methoxyphenol vollständig gelöst hat. Anschließend werden 2,2g Schwefelsäure zugegeben und der Ansatz wird bei einem Druck von 800 mbar auf 90°C aufgeheizt. Dabei wird ein gleichmäßiger Luftstrom über eine Gasfritte in den Ansatz eingeleitet. Sobald 90°C erreicht sind, wird das Vakuum auf 400 mbar verschärft, woraufhin der Ansatz anfängt zu destillieren. Nach ca 3-4 Stunden versiegt der Destillatfluß und das Vakuum wird zur Vervollständigung der Reaktion auf 100 mbar verbessert. Nach weiteren 2 Stunden wird der Ansatz abgekühlt und ausgefüllt. Erhalten werden 451 g eines hellgelben Öls. Säurezahl: 69,1 mg KOH/g, Verseifungszahl: 175,6 mg KOH/g, Veresterungsgrad: 99%.

### Beispiel 5 (Acrylsäureesters von 5-EO-Allylalkohol, AAA5):

275 g (1 mol) 5 EO Allylalkohol aus Beispiel 1, 0,8 g p-Methoxyphenol, 216,3 g (3 mol) Acrylsäure und 2,0 g Schwefelsäure werden wie im Beispiel 4 umgesetzt. Man erhält 385 g gelbes Öl. Säurezahl: 76,5 mg KOH/g; Verseifungszahl: 237,4 mg KOH/g. Veresterungsgrad 94,5%

### Beispiel 6 (Acrylsäureesters von 20 EO-Allylalkohol, AAA20):

445,3 g (0,42 mol) 20-EO-Allylalkohol aus Beispiel 3, 0,8 g p-Methoxyphenol, 216,4 g (3 mol) Acrylsäure und 2,0 g Schwefelsäure werden wie im Beispiel 4 umgesetzt. Man erhält 547 g eines gelbroten Wachses. Säurezahl 68,5 mg KOH/g; Verseifungszahl: 126,1 mg KOH/g. Veresterungsgrad 97%.

### Beispiel 7 ( Methacrylsäureester von 20-EO-Allylalkohol, MAA20):

445,3 g (0,42 mol) 20-EO-Allylalkohol aus Beispiel 3, 0,8 g p-Methoxyphenol, 385 g (4,53 mol) Methacrylsäure und 4 g konz. Schwefelsäure werden analog dem Beispiel 4 umgesetzt, wobei die Reaktionszeit verdoppelt und das Vakuum am Ende der Reaktion auf 20 mbar verschärft wird. Man erhält 555 g festes Produkt. Säurezahl 103,5 mg KOH/g; Verseifungszahl 158,3 mg KOH/g; Veresterungsgrad 93,9%.

### Beispiel 8 (Methacrylsäureester von 5 EO-Allylalkohol,MAA5):

275 g (1 mol) 5-EO-Allylalkohol aus Beispiel 1 werden zusammen mit 0,8 g p-Methoxyphenol, 516,6 g Methacrylsäure (6,08 mol) und 2 g konz. Schwefelsäure analog Beispiel 7 umgesetzt. Erhalten wird eine gelbe Flüssigkeit. Säurezahl: 98,1 mg KOH/g; Verseifungszahl: 247,8 mg KOH/g; Veresterungsgrad 91,6%.

### Beispiel 9 (Methacrylsäureester von 10-EO-Allylalkohol,MAA10):

479,5 g (1,01 mol) 10-EO-Allylalkohol aus Beispiel 2, 0,8 g p-Methoxyphenol, 258,3 g (3,04 mol) Methacrylsäure werden zusammen wie im Beispiel 7 umgesetzt. Erhalten werden 680 g eines gelben Öls. Säurezahl: 99,5 mg KOH/g; Verseifungszahl: 196,0 mg KOH/g; Veresterungsgrad 94%.

Die nachfolgend beschriebenen Polymerisationsansätze (**Beispiele 10 - 17** und **Vergleichsbeispiele V1 - V5** werden nach folgender allgemeinen Herstellungsweise durchgeführt:

### a) Vorprodukt

In einem zylindrischen Kunststoffgefäß wird eine Monomerlösung aus 265,2 g Acrylsäure und 372,4 g vollentsalztem Wasser sowie der verwendeten Vernetzer angesetzt. Unter Rühren und Kühlen wird mit 206,1 g 50%-iger Natronlauge (70% Neutralisationsgrad) teilneutralisiert. Die Lösung wird auf 7-8°C abgekühlt und mit Stickstoff solange durchperlt, bis der Sierstoffgehalt in der Monomerenlösung auf einen Wert von unter 0,2 ppm abgesunken ist. Anschließend gibt man 0,3 g Azo-bis (2-amodinopropan) dihydrochlorid gelöst in 10 g voll entnetztem-Wasser, 0,05 g Natriumpersulfat, gelöst in 6 g VE-Wasser, 0,005 g Wasserstoffperoxid (35%-ig) gelöst in 1 g VE-Wasser und 2g Natriumcarbonat hinzu. Danach wird die Polymerisation durch Zugabe von 0,012 g Ascorbinsäure gelöst in 2g Wasser gestartet, worauf eine deutliche Temperaturerhöhung festgestellt wird. Das Polymere wird anschließend gewölft, bei 140°C im Umlufttrockenschrank getrocknet, gemahlen und auf die Kornfraktion 150-850 µm abgesiebt.

Das **Beispiel 18** und die **Vergleichsbeispiele V6 - V10** wurden nach der zuvor genannten allgemeinen Herstellungsvorschrift polymerisiert, jedoch ohne den Zusatz von Natriumcarbonat.

### b) Nachvernetzung:

100 g des gemahlenen und auf 150 - 800 mm abgesiebten Polymeren werden unter kräftigem Durchmischen mit einer Lösung aus 0,5 g Ethylencarbonat und 1,5 g VE-Wasser in einem Mischer der Firma MTI benetzt und anschließend für 30 Minuten in einem Ofen auf eine Temperatur von 180°C erhitzt.

Die Tabelle im Anhang 1 zeigt die Zusammensetzung und die Eigenschaften der Polymerisate gemäß der Beispiele 10 - 18, sowie der Vergleichsbeispiele V1 - V 10.

Man erkennt aus der Tabelle, daß die erfindungsgemäßen Beispiele 10 - 18 Polymerisate mit einer Kombination guter Eigenschaften liefern:

| | |
|---|---|
| Retention | >30 g/g und |
| Absorption unter Druck (21 g/cm²) | >30 g/g und |
| Absorption unter Druck (49 g/cm²) | >20 g/g und |
| Schwellgeschwindigkeit | <40 s und |
| Lösliche Anteile (16h) | <12% |

Bei den Vergleichsbeispielen ist es zwar möglich, durch Variation der Vernetzermenge einzelne, aber nicht die Kombination dieser guten Eigenschaften zu erreichen.

So können mit dem Craynor 435-Vernetzer (entsprechend WO 93/21237 und WO 94/9043) je nach Einsatzmenge (V2 und V4) entweder Produkte mit einem guten AUP (49 g/cm²) von 26,7 g/g und noch akzeptablen löslichen Anteilen, dafür aber mit schlechter Retention erhalten werden oder aber ein Produkt mit guter Retention und vertretbarem AUP, dafür aber schlechten löslichen Anteilen. Die Produkte mit nur einem Vernetzer weisen trotz Zusatz von Natriumcarbonat durchweg eine schlechte Schwellgeschwindigkeit auf.

Die Vernetzerkombination aus Trimethylolpropantriacrylat und Triallylamin des Vergleichsbeispiels V6 wird in der US 5 314 420 als eine zu bevorzugende Kombination angegeben. Aus den Ergebnissen erkennt man, daß weder die Retention, noch die Schwellgeschwindigkeit, noch die löslichen Anteile die Eigenschaften der erfindungsgemäßen Polymerisate erreichen.

Aus den Versuchen ohne Zusatz von Natriumcarbonat (Bsp. 18, V8 - V10) erkennt man, daß die erfindungsgemäßen Polymerisate ihr hervorragendes Eigerschaftsprofil beibehalten, d.h. die Verbesserungen bei der Schwellgeschwindigkeit sind gegenüber dem Stand der Technik unabhängig von der Verwendung dieser Komponente. Die Vergleichsbeispiele zeigen ohne den Zusatz von Natriumcarbonat schlechtere Eigenschaften.

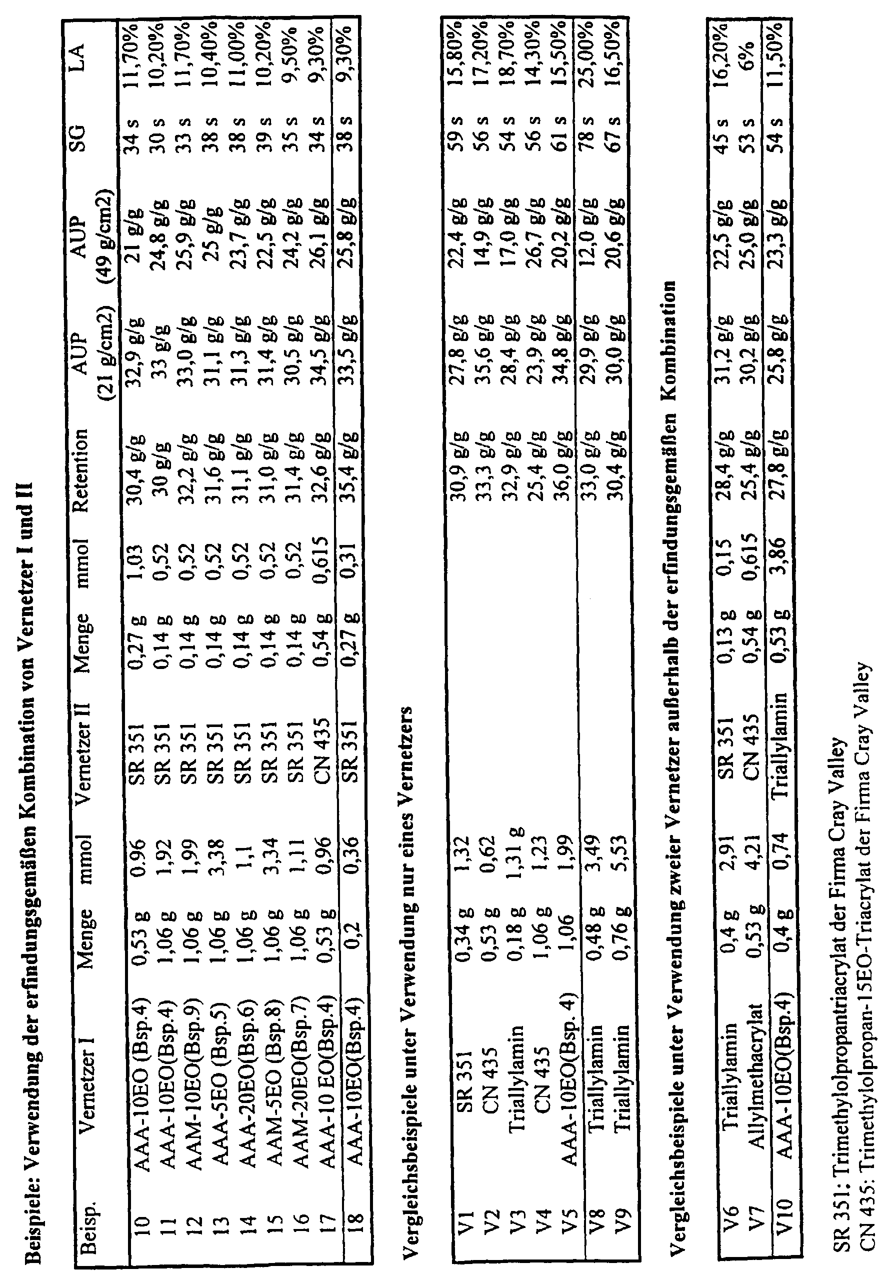

## Patentansprüche

1. Wäßrige Flüssigkciten absorbierendes vernetztes Polymerisat, aufgebaut aus teilneutralisierten monoethylenisch ungesättigten Säuregruppen tragenden Monomeren, gegebenenfalls weiteren damit copolymerisierbaren Monomeren und gegebenenfalls als Pfropfgrundlage geeigneten Polymeren dadurch gekennzeichnet, daß es unter Verwendung einer Vernetzerkombination aus
I. 40-90 Mol% CH₂=CR⁵-CO-(OCHR³-CHR³)_{z}O-CH₂-CR⁵=CH₂
II. 10-60 Mol% R¹-[O(CHR³-CHR³O)ᵤ-CO-R²]ₓ
mit
R¹: mehrwertiges C₂₋₁₀-Alkyl,
R²: linear oder verzweigt C₂₋₁₀-Alkenyl,
R³: H, CH₃, C₂H₅,
R⁵: H, CH₃,
x: 2-6,
u: 0-15,
z: 3-20
und einem Nachvernetzer herstellbar ist.

2. Polymerisat nach Anspruch 1, dadurch gekennzeichnet, daß der Vernetzer I zu 50-80 Mol % und der Vernetzer II zu 20-50 Mol % bezogen auf die Vernetzermischung eingesetzt wird.

3. Polymerisat nach einem der Ansprüche 1-2, dadurch gekennzeichnet, daß der Vernetzer I zu 60-80 Mol % und der Vernetzer II zu 20-40 Mol % bezogen auf die Vernetzermischung eingesetzt wird.

4. Polymerisat nach einem der Ansprüche 1-3, dadurch gekennzeichnet, daß die Vernetzerkombination in einer Menge von 0,1 - 2,0 Gew. % bezogen auf die Monomeren eingesetzt werden.

5. Polymerisat nach einem der Ansprüche 1-4, dadurch gekennzeichnet, daß die Vernetzerkombination in einer Menge von 0,3 - 1,0 Gew. % bezogen auf die Monomeren eingesetzt werden.

6. Polymerisat nach einem der Ansprüche 1 bis 5, dadurch gekennzeichnet, daß der Vernetzer I in der Polyglykolkette im Gewichtsmittel aus mindestens 3, bevorzugt 5-20 und besonders bevorzugt aus 8-12 Glykoleinheiten besteht.

7. Polymerisat nach einem der Ansprüche 1 bis 6, dadurch gekennzeichnet, daß die ungesättigten Säuregruppen tragenden Monomeren aus der Gruppe Acrylsäure, Methacrylsäure, Vinylessigsäure, Vinylsulfonsäure, Methallylsulfonsäure und 2-Acrylamido-2-methylpropansulfonsäure ausgewählt sind.

8. Polymerisat nach einem der Ansprüche 1 bis 7, dadurch gekennzeichnet, daß es 0 bis 40 Gew. %, bezogen auf die säuregruppenenthaltenden Monomeren weitere Comonomere aus der Gruppe (Meth)acrylamid, (Meth)acrylnitril, Vinylpyrrolidon, Hydroxyethylacrylat und Vinylacetamid einpolymerisiert enthält.

9. Polymerisat nach einem der Ansprüche 1 bis 8, dadurch gekennzeichnet, daß es 0 bis 30 Gew.%, bezogen auf die Summe aller Monomeren an als Pfropfgrundlage geeigneten wasserlöslichen Polymeren, vorzugsweise Polysaccharide und/oder Polyvinylalkohol enthält.

10. Polymerisat nach einem der Ansprüche 1 bis 9, dadurch gekennzeichnet, daß die Nachvernetzung gegebenenfalls mehrfach wiederholt wird.

11. Polymerisat gemäß Anspruch 10, dadurch gekennzeichnet, daß es an der Oberfläche mit einem Nachvernetzer aus der Gruppe der Polyole, Polyepoxide, Polyamine oder Alkylencarbonate vernetzt wurde.

12. Polymerisat nach einem der Ansprüche 10 und 11, dadurch gekennzeichnet, daß es eine Retention von mindestens 30 g/g, eine Flüssigkeitaufnahme unter Druck (49 g/cm2 ) von mindestens 20 g/g, lösliche Anteile nach 16 Stunden von höchstens 12 % und eine Schwellgeschwindigkeit von unter 40 sec aufweist.

13. Polymerisat nach einem der Ansprüche 10, 11 und 12, dadurch gekennzeichnet, daß es eine Flüssigkeitsaufnahme unter Druck (49 g/cm²) von mindestens 22 g/g aufweist.

14. Polymerisat nach einem der Ansprüche 10, 11 und 12, dadurch gekennzeichnet, daß es eine Schwellgeschwindigkeit von unter 35 sec aufweist.

15. Polymerisat nach einem der Ansprüche 10, 11 und 12, dadurch gekennzeichnet, daß die löslichen Anteile nach 16 Stunden höchstens 10 % betragen.

16. Verfahren zur Herstellung eines wäßrige Flüssigkeiten absorbierenden, vernetzten Polymerisates nach einem der Ansprüche 1 bis 9, dadurch gekennzeichnet, daß eine wäßrige Lösung aus ungesättigten, Säuregruppen tragenden, teilneutralisierten Monomeren und einer Vernetzermischung aus
I. 40-90 Mol% CH₂=CR⁵-CO-(OCHR³-CHR³)_{z}O-CH₂-CR⁵=CH₂
II. 10-60 Mol% R¹-[O(CHR³-CHR³O)ᵤ-CO-R²]ₓ
mit
R¹: mehrwertiges C₂₋₁₀-Alkyl,
R²: linear oder verzweigt C₂₋₁₀-Alkenyl,
R³: H, CH₃, C₂H₅,
R⁵: H, CH₃,
x: 2-6,
u: 0-15,
z: 3-20
unter Zusatz von Radikalbildnern nach dem Verfahren einer Lösungs- oder Suspensionspolymerisation zu einem Hydrogel polymerisiert, zerkleinert, getrocknet, gemahlen und gesiebt wird, die Polymerisate mit einem Oberflächenvernetzer behandelt werden und Oberflächenvernetzung bei erhöhter Temperatur durchgeführt wird.

17. Verfahren nach Anspruch 16, dadurch gekennzeichnet, daß die Oberflächenbehandlung und Vernetzung mehrmals durchgeführt wird.

18. Verwendung der Polymerisate nach einem der Ansprüche 1 bis 15 als Absorptionsmittel für Wasser und wäßrige Flüssigkeiten, insbesondere in Konstruktionen zur Aufnahme von Körperflussigkeiten, in geschäumten oder nicht geschäumten Flächengebilden, in Strom- oder lichtleitenden Kabeln, in Verpackungsmaterialien, in Bodenverbesserungsmitteln, bei der Pflanzenaufzucht und als Träger für Düngemittel oder andere Wirkstoffe, die über einen längeren Zeitraum wieder verzögert an die Umgebung abgegeben werden.

## Claims

1. A crosslinked polymer product which absorbs aqueous liquids and is constituted of partially neutralized, monoethylenically unsaturated monomers bearing acid groups, optionally other monomers copolymerizable there-with, and optionally polymers suitable as a basis for grafting, characterized in that the polymer product can be produced using a crosslinker combination of
I. 40-90 mole-% of CH₂=CR⁵-CO(OCHR³-CHR³)_{z}O-CH₂-CR⁵=CH₂
II. 10-60 mole-% of R¹-[O(CHR³-CHR³O)ᵤ-CO-R²]ₓ,
wherein
R¹: multivalent C₂₋₁₀ alkyl,
R²: linear or branched C₂₋₁₀ alkenyl,
R³: H, CH₃, C₂H₅,
R⁵: H, CH₃,
x: 2-6,
u: 0-15,
z: 3-20,
and a secondary crosslinker.

2. The polymer product of claim 1, characterized in that the crosslinker I is employed with 50-80 mole-%, and the crosslinker II with 20-50 mole-%, relative to the cross linker mixture.

3. The polymer product according to claims 1-2, characterized in that the crosslinker I is employed with 60-80 mole-% and the crosslinker II with 20-40 mole-%, relative to the crosslinker mixture.

4. The polymer product according to any of claims 1-3, characterized in that the crosslinker combination is employed in an amount of from 0.1-2.0 wt.-%, relative to the monomers.

5. The polymer product according to any of claims 1-4, characterized in that the crosslinker combination is employed in an amount of from 0.3-1.0 wt.-%, relative to the monomers.

6. The polymer product according to any of claims 1-5, characterized in that on a weight average, the crosslinker I in its polyglycol chain consists of at least 3, preferably 5-20, and more preferably 8-12 glycol units.

7. The polymer product according to any of claims 1-6, characterized in that the unsaturated monomers bearing acid groups are selected from the group of acrylic acid, methacrylic acid, vinylacetic acid, vinylsulfonic acid, methallylsulfonic acid, and 2-acrylamido-2-methyl-propanesulfonic acid.

8. The polymer product according to any of claims 1-7, characterized in that it contains polymerized there-in from 0 to 40 wt.-% of other comonomers from the group of (meth)acrylamide, (meth)acrylonitrile, vinylpyrrolidone, hydroxyethyl acrylate, and vinylacetamide, relative to the monomers containing acid groups.

9. The polymer product according to any of claims 1-8, characterized in that it contains, relative to the overall monomers, from 0 to 30 wt.-% of water-soluble polymers suitable as a basis for grafting, preferably polysaccharides and/or polyvinyl alcohol.

10. The polymer product according to any of claims 1-9, characterized in that the secondary crosslinking is optionally repeated several times.

11. The polymer product according to claim 10, characterized in that it has been crosslinked on its surface using a secondary crosslinker from the group of polyols, polyepoxides, polyamines, or alkylene carbonates.

12. The polymer product according to any of clams 10 and 11, characterized by a retention of at least 30 g/g, a liquid absorption under pressure (49 g/cm²)of at least 20 g/g, solubles after 16 hours of 12% at maximum, and a swelling rate of below 40 s.

13. The polymer product according to any of claims 10, 11 and 12, characterized by a liquid absorption under pressure (49 g/cm²) of at least 22 g/g.

14. The polymer product according to any of claims 10, 11 and 12, characterized by a swelling rate of below 35 s.

15. The polymer product according to any of claims 10, 11 and 12, characterized in that the solubles are 10% at maximum after 16 hours.

16. A process for producing an aqueous liquid-absorbing, crosslinked polymer product according to any of claims 1 through 9, characterized in that an aqueous solution of unsaturated, partially neutralized monomers bearing acid groups, and a mixture of monomers, comprising
I. 40-90 mole-% of CH₂=CR⁵-CO(OCHR³-CHR³)_{z}O-CH₂CR⁵=CH₂
II. 10-60 mole-% of R¹-[O(CHR³-CHR³O)ᵤ-CO-R²]ₓ,
wherein
R¹: multivalent C₂₋₁₀ alkyl,
R²: linear or branched C₂₋₁₀ alkenyl,
R³: H, CH₃, C₂H₅,
R⁵: H, CH₃,
x: 2-6,
u: 0-15,
z: 3-20,
is polymerized with addition of free radical formers according to the procedure of a solution or suspension polymerization to form a hydrogel which is crushed, dried, milled, and screened, the polymer products are treated with a surface crosslinker, and surface cross-linking is performed at elevated temperature.

17. The process according to claim 16, characterized in that surface treatment and crosslinking are performed several times.

18. Use of the polymer products according to any of claims 1 through 15 as an absorbent for water and aqueous liquids, particularly in constructions for absorbing body fluids, in foamed or non-foamed sheet materials, in electroconductive or light-conducting cables, in packaging materials, in soil improvers, in plant breeding, and as a supporting material for fertilizers or other active ingredients which are released into the environment in a delayed fashion over a prolonged period of time.

## Revendications

1. Polymère absorbant les liquides aqueux, constitué de monomères portant des groupes d'acide monoéthyléniquement insaturé partiellement neutralisés, éventuellement d'autres monomères copolymérisables avec eux, et éventuellement des polymères convenant comme base de greffage, caractérisé en ce qu'il peut être préparé en utilisant une combinaison d'agents réticulants constituée de:
I. 40 à 90% molaires de CH₂=CR⁵-CO-(OCHR³-CHR³)_{z}O-CH₂CR⁵=CH₂
II. 10 à 60% molaires de R¹-[O(CHR³-CHR³O)ᵤ-CO-R²]ₓ
où
R¹ représente un radical alkyle polyvalent en C₂-C₁₀,
R² représente un radical alcényle linéaire ou ramifié en C₂ à C₁₀,
R³ représente H, CH₃, C₂H₅,
R⁵ représente H, CH₃,
x a une valeur de 2 à 6,
u a une valeur de 0 à 15,
z a une valeur de 3 à 20,
et un agent post-réticulant.

2. Polymère selon la revendication 1, caractérisé en ce que l'agent réticulant I est mis en oeuvre à raison de 50 à 80% molaires et l'agent de réticulation II à raison de 20 à 50% molaires, par rapport au mélange d'agents réticulants.

3. Polymère selon l'une des revendications 1 ou 2, caractérisé en ce que l'agent réticulant I est mis en oeuvre à raison de 60 à 80% molaires et l'agent de réticulation II à raison de 20 à 40% molaires, par rapport au mélange d'agents réticulants.

4. Polymère selon l'une des revendications 1 à 3, caractérisé en ce que la combinaison d'agents réticulants est mise en oeuvre en quantités de 0,1 à 0,2% en poids, par rapport aux monomères.

5. Polymère selon l'une des revendications 1 à 4, caractérisé en ce que la combinaison d'agents réticulants est mise en oeuvre en quantités de 0,3 à 1,0% en poids, par rapport aux monomères

6. Polymère selon l'une des revendications 1 à 5, caractérisé en ce que l'agent de réticulation I est constitué d'unités de glycol dans une moyenne pondérale de 3, de préférence de 5 à 20 et avantageusement de 8 à 12 dans la chaîne glycolique.

7. Polymère selon l'une des revendications 1 à 6, caractérisé en ce que les monomères portant des groupes d'acide insaturé sont choisis dans le groupe formé par l'acide acrylique, l'acide méthacrylique, l'acide vinylacétique, l'acide vinylsulfonique, l'acide méthallylsulfonique et l'acide 2-acrylamido-2-méthylpropane-sulfonique.

8. Polymère selon l'une des revendications 1 à 7, caractérisé en ce qu'il contient de 0 à 40% en poids, par rapport aux monomères contenant des groupes acides, d'autres comonomères du groupe formé par le (méth)acrylamide, le (méth)acrylonitrile, la vinylpyrrolidone, l'acrylate d'hydroxyéthyle et le vinylacétamide polymérisés avec lui.

9. Polymére selon l'une des revendications 1 à 8, caractérisé en ce qu'il contient de 0 à 30% en poids, par rapport à la somme de tous les monomères, d'un polymère soluble dans l'eau convenant comme base de greffage, de préférence des polysaccharides et/ou du poly(alcool vinylique).

10. Polymère selon l'une des revendications 1 à 9, caractérisé en ce que la post-réticulation est éventuellement répétée plusieurs fois.

11. Polymère selon la revendication 10, caractérisé en ce qu'il a été réticulé en surface par un agent de post-réticulation du groupe formé par les polyols, les polyépoxydes, les polyamines ou des carbonates d'alkyle.

12. Polymère selon l'une des revendications 10 ou 11, caractérisé en ce qu'il présente une rétention d'au moins 30 g/g, une absorption de liquide sous pression (49 g/cm²) d'au moins 20 g/g, une fraction soluble après 16 heures d'un maximum de 12% et une vitesse de gonflement de moins de 40 secondes.

13. Polymère selon l'une des revendications 10, 11 et 12, caractérisé en ce qu'il présente une absorption de liquide sous pression (49 g/cm²) d'au moins 22 g/g.

14. Polymère selon l'une des revendications 10, 11 et 12, caractérisé en ce qu'il présente une vitesse de gonflement de moins de 35 secondes.

15. Polymère selon l'une des revendications 10, 11 et 12, caractérisé en ce que les fractions solides après 16 heures représentent 10% au maximum.

16. Procédé de préparation d'un polymère réticulé absorbant les liquides aqueux selon l'une des revendications 1 à 9, caractérisé en ce que l'on polymérise une solution aqueuse de monomères insaturés portant des groupes acides, partiellement neutralisés, et d'un mélange d'agents réticulants constitué de
I. 40 à 90% molaires de CH₂=CR⁵-CO-(OCHR³-CHR³)_{z}O-CH₂-CR⁵=CH₂
II. 10 à 60% molaires de R¹-[O(CHR³-CHR³O)ᵤ-CO-R²]ₓ
où
R¹ représente un radical alkyle polyvalent en C₂-C₁₀,
R² représente un radical alcényle linéaire ou ramifié en C₂ à C₁₀,
R³ représente H, CH₃, C₂H₅,
R⁵ représente H, CH₃,
x a une valeur de 2 à 6,
u a une valeur de 0 à 15,
z a une valeur de 3 à 20,
en utilisant des médiateurs radicalaires, selon le procédé de polymérisation en solution ou en suspension, de manière à former un hydrogel, que l'on procède ensuite à un broyage, un séchage et un criblage, que les polymères sont traités par un agent réticulant de surface et que la réticulation superficielle est entreprise à température élevée.

17. Procédé selon la revendication 16, caractérisé en ce que le traitement de surface et la réticulation sont effectués plusieurs fois.

18. Utilisation des polymères selon l'une des revendications 1 à 15 en tant que matière absorbante pour l'eau et les liquides aqueux, en particulier dans des systèmes destinés à recueillir des liquides corporels, dans des produits plats expansés ou non expanses, des câbles conducteurs ou d'éclairage, des matériaux d'emballage, des systèmes d'assainissement des sols, dans la culture de plantes et en tant que support pour engrais ou autres agents qui sont restitués à retardement et à long terme dans l'environnement.
